# EUROPÄISCHE PATENTSCHRIFT

(11) **EP 1 786 776 B1**
(45) Veröffentlichungstag und Bekanntmachung des Hinweises auf die Patenterteilung: **03.12.2008**
(21) Anmeldenummer: 05777970.4
(22) Anmeldetag: 03.08.2005
(51) Int. Cl.: C07D 213/20, C07D 233/54, C07C 209/20

(54) **VERFAHREN ZUR HERSTELLUNG QUARTÄRER AMMONIUMVERBINDUNGEN HOHER REINHEIT**
METHOD FOR PRODUCING HIGH-PURITY QUATERNARY AMMONIUM COMPOUNDS
PROCEDE DE PRODUCTION DE COMPOSES D'AMMONIUM QUATERNAIRES D'UNE GRANDE PURETE

(30) Priorität: 24.08.2004 DE 102004041126
(43) Veröffentlichungstag der Anmeldung: 23.05.2007
(73) Patentinhaber: BASF SE, 67056 Ludwigshafen (DE)
(72) Erfinder: SZARVAS, Laszlo, 67071 Ludwigshafen (DE); MASSONNE, Klemens, 67098 Bad Dürkheim (DE)
(86) Internationale Anmeldenummer: PCT/EP2005/008411
(87) Internationale Veröffentlichungsnummer: WO 2006/021302

(56) Entgegenhaltungen:
- DE-C- 228 247
- US-A- 2 583 772
- DATABASE WPI Section Ch, Week 200245 Derwent Publications Ltd., London, GB; Class E19, AN 2002-419496 XP002355160 & JP 2001 322970 A (TONEN KAGAKU KK) 20. November 2001 (2001-11-20) in der Anmeldung erwähnt
- VOSS W ET AL: "UEBER DIE EINWIRKUNG VON SCHWEFLIGSAEURE-ESTERN AUF AMINOSAEUREN. IV. MITTEIL UEBER ESTER SER SCHWEFLIGEN SAEURE ESTERS OF SULFUROUS ACID. IV. ACTION OF SULFUROUS ESTERS ON AMINO ACIDS" BER, XX, XX, Bd. 70, 1937, Seiten 388-392, XP008052546
- VOSS W ET AL: "UEBER DIE ESTER DER SCHWEFLIGEN SAEURE. I" JUSTUS LIEBIGS ANNALEN DER CHEMIE, VERLAG CHEMIE, WEINHEIM,, DE, Bd. 485, 1931, Seiten 258-283, XP000913973 ISSN: 0075-4617

## Beschreibung

Die vorliegende Erfindung betrifft ein Verfahren zur Herstellung quartärer Ammoniumverbindungen durch Umsetzung des entsprechenden tertiären sp³-hybridisierten Amins oder sp²-hybridisierten Imins mit Dimethylsulfit.

Quartäre Ammoniumverbindungen sind wichtige Substanzen, welche eine breite Anwendung finden. So werden diese beispielsweise eingesetzt als Weichspüler-Wirkstoffe, in der Hygiene und Kosmetik, als Phasentransfer-Katalysatoren oder als Leitsalze für elektronische Anwendungen. Eine weitere wichtige Anwendungsgruppe sind ionische Flüssigkeiten mit Alkylammonium, Imidazolium oder Pyridinium als Kationen.

Quartäre Ammoniumverbindungen mit mindestens einer Methylgruppe am Stickstoff und frei wählbarem Anion werden üblicherweise in einer Zweistufenreaktion hergestellt. In der ersten Synthesestufe wird das entsprechende tertiäre Amin/Imin mit einem Methylierungsmittel methyliert, wobei das erhaltene Anion der quartären Ammoniumverbindung durch das eingesetzte Methylierungsmittel festgelegt ist. Um das gewünschte Anion einzuführen wird anschließend in der zweiten Synthesestufe ein sogenannter Anionentausch durchgeführt.

Die Methylierung (erste Synthesestufe) erfolgt üblicherweise durch Umsetzung der entsprechenden tertiären Amine/Imine mit Methylierungsmitteln.

Die üblicherweise eingesetzten Methylierungsmittel sind die Methylester starker Mineralsäuren wie insbesondere Dimethylsulfat oder Methylchlorid (siehe z.B. Houben-Weyl, Methoden der organischen Chemie, 4. Auflage, Band XI/2, Georg Thieme Verlag, Stuttgart 1958, Seite 591 bis 630). Nachteilig am Einsatz von Dimethylsulfat ist seine carcinogene Wirkung, welche ein Gefährdungspotenzial darstellt und aufwändige Sicherheitsmaßnahmen erfordert. Nachteilig am Einsatz von Methylchlorid ist seine Reaktionsträgheit und damit verbunden eine erhöhte Reaktionstemperatur sowie ein erhöhter Reaktionsdruck. Dadurch finden Nebenreaktionen statt, welche die Aufarbeitung erschweren und die Ausbeute verringern.

Alternativ ist in JP 04-341,593 und JP 09-025,173 der Einsatz von Dimethylcarbonat als Methylierungsmittel beschrieben. Nachteilig an diesem ist seine Reaktionsträgheit und damit verbunden eine erhöhte Reaktionstemperatur von über 100°C sowie ein erhöhter Reaktionsdruck von etwa 1 bis 4 MPa abs. Dadurch finden Nebenreaktionen statt, welche die Aufarbeitung erschweren und die Ausbeute verringern. So wird beispielsweise bei der Methylierung von Imidazol unter diesen Bedingungen der Kern carboxyliert. Beim Einsatz von tertiären Alkylaminen als Edukt findet unter diesen Bedingungen der Hoffmann-Abbau statt.

Des Weiteren ist auch Methyliodid als Methylierungsmittel zur Darstellung quartärer Ammoniumverbindungen bekannt. Nachteilig am Einsatz von Methyliodid ist jedoch seine carcinogene Wirkung, welche ein Gefährdungspotenzial darstellt und aufwändige Sicherheitsmaßnahmen erfordert. Ferner ist Methyliodid in den erforderlichen technischen Mengen nicht verfügbar beziehungsweise gegenüber den oben genannten Methylierungsmitteln relativ teuer.

Auch der Einsatz von Dimethylsulfit als Methylierungsmittel zur Darstellung quartärer Ammoniumverbindungen ist an sich bekannt. So ist in der DE-Patentschrift 228 247 die Umsetzung verschiedener Alkaloide der Morphinreihe mit Dimethylsulfit in Gegenwart von Methanol als Lösungsmittel durch Erhitzen im Wasserbad zu den entsprechenden Morphinium-methylsulfiten (nach der in der DE-Schrift verwendeten alten Nomenklatur "methylatsulfiten") beschrieben. Die Isolierung der Morphinium-methylsulfite erfolgte durch Abdestillation des Lösungsmittels und überschüssigen Dimethylsulfits im Vakuum und anschließender Trocknung. DE 228 247 offenbart des Weiteren die anschließende Umsetzung der erhaltenen Morphinium-methylsulfite mit Metallhalogeniden oder Halogenwasserstoffsäuren zu den entsprechenden Morphinium-halogeniden.

JP 2001-322,970 beschreibt die Umsetzung von aliphatischen Trialkylaminen mit Dimethylsulfit in Gegenwart eines polaren Lösungsmittels wie beispielsweise einem Alkohol oder Acetonitril bei 40 bis 100°C zu den entsprechenden Methyl-trialkylammonium-methylsulfiten. Die Isolierung des Produkts erfolgte durch Abdestillation des Lösungsmittels im Vakuum. JP 2001-322,970 offenbart des Weiteren zur Einführung des gewünschten Anions die anschließende Umsetzung der erhaltenen Methyltrialkylammonium-methylsulfite mit wässriger Säure.

Dimethylsulfit besitzt gegenüber den anderen, oben aufgezählten Methylierungsmitteln den großen Vorteil einer ausreichenden Methylierungsstärke, was schonende Reaktionsbedingungen ermöglicht, und zugleich der relativ leichten Entfembarkeit des Großteils des Methylsulfit-Anions nach Zugabe der Säure des gewünschten Anions durch Erhitzen unter Bildung von Methanol und flüchtigem Schwefeldioxid. Erfindungsgemäß wurde jedoch erkannt, dass durch die in DE 228 247 und JP 2001-322,970 beschriebenen Verfahren dennoch ein Schwefel-Gehalt in der Größenordnung von ≥ 2 Gew.-% in der isolierten quartären Ammoniumverbindung nach Umsetzung mit der Säure des gewünschten Anions verbleibt. Dieser Schwefel-Gehalt stört jedoch bei verschiedenen Anwendungen der quartären Ammoniumverbindung, insbesondere bei deren Einsatz in der Elektronikindustrie. Die nach den im Stand der Technik beschriebenen Verfahren hergestellten quartären Ammoniumverbindungen müssen daher vor deren Einsatz erst aufwändig gereinigt werden, was einen entscheidenden Nachteil darstellt.

Der Anionentausch (zweite Synthesestufe) erfolgt üblicherweise durch Umsetzung mit
(i) der Säure des gewünschten Anions, insbesondere wenn das ursprünglich in der Methylierung eingeführte Anion in leicht abtrennbare Produkte zersetzbar ist (z.B. Methylcarbonat, Methylsulfit); oder wenn zwischen dem quartären Ammoniumsalz des ursprünglich in der Methylierung eingeführten Anions und quartären Ammoniumsalz des gewünschten Anions ein signifikanter Unterschied in der Löslichkeit in einem bestimmten Lösungsmittel oder der Kristallisierbarkeit besteht;
(ii) einer Verbindung, welche mit dem ursprünglich in der Methylierung eingeführten Anion oder einem ausgetauschten Anion reagiert und dabei das gewünschte Anion bildet (z.B. Austausch von Cl⁻ gegen F via KF-Zugabe und Phasentransferkatalyse und Umsetzung des F mit BF₃·OMe₂ zur Bildung von [BF₄]⁻; oder Umsetzung von Br⁻ mit Me-SO₂-OC₃H₇ zur Bildung von Me-SO₃⁻);
(iii) einem Metallsalz des gewünschten Anions, insbesondere wenn das Metallsalz des ursprünglich in der Methylierung eingeführten Anions sehr schlecht löslich ist (z.B. Ausfall von Silberchlorid) oder in einem hydrophilen/hydrophoben ZweiPhasensystem eine signifikante Anreicherung des quartären Ammoniumsalzes mit dem gewünschten Anion in einer Phase möglich ist (lonenpaar-Extraktion);
(iv) Silberhydroxid, falls das ursprünglich in der Methylierung eingeführte Anion Chlorid, Bromid oder lodid ist, zur Einführung des Hydroxid-Anions, welches anschließend mit der Säure des gewünschten Anions neutralisiert werden kann;
(v) einem unlöslichen (polymeren) Anionentauscher.

Eine Übersicht über verschiedene Varianten des Anionenaustauschs ist in Houben-Weyl, Methoden der organischen Chemie, 4. Auflage, Band XI/2, Georg Thieme Verlag, Stuttgart 1958, Seite 591 bis 630 und Houben-Weyl, Methoden der organischen Chemie, Erweiterungs- und Folgebände zur 4. Auflage, Band E16a, Teil 2, Georg Thieme Verlag, Stuttgart 1990, Seite 997 bis 1017 gegeben.

Nachteilig am Anionentausch ist die mindestens zweistufige Synthese, welche einen hohen technischen Aufwand bedingt und nicht zuletzt auch wegen der Isolierung der Zwischenstufe nur eine verminderte Ausbeute ermöglicht. Ferner ist bei den oben genannten Methoden (iii) und (iv) auch der erforderliche Umgang mit Feststoffen von Nachteil. Des Weiteren weist die durch Anionentausch erhältliche quartäre Ammoniumverbindung im Allgemeinen nicht die, für einen Einsatz in der Elektronikindustrie erforderliche hohe Reinheit auf, so dass diese üblicherweise einer aufwändigen Nachreinigung unterzogen werden muss.

Aufgabe der vorliegenden Erfindung war es, ein Verfahren zur Herstellung quartärer Ammoniumverbindungen zu finden, welches die Nachteile des Standes der Technik nicht aufweist, einfach durchzuführen ist, das einzusetzende Alkylierungsmittel nicht oder nur geringfügig toxisch ist und eine einfache und flexible Einführung des gewünschten Anions ermöglicht, wobei die quartäre Ammoniumverbindung mit dem gewünschten Anion in hoher Reinheit und hoher Ausbeute ohne aufwändige Reinigungsschritte darstellbar und auch für den Einsatz in der Elektronikindustrie geeignet sein soll.

Demgemäß wurde ein Verfahren zur Herstellung quartärer Ammoniumverbindungen durch Umsetzung des entsprechenden tertiären sp³-hybridisierten Amins oder sp²-hybridisierten Imins mit Dimethylsulfit gefunden, das dadurch gekennzeichnet ist, dass man die Umsetzung
(i) in Gegenwart einer anorganischen oder organischen Protonensäure mit einem pKₐ-Wert von 1,8 bis 14, gemessen bei 25°C in wässriger Lösung; und
(ii) bei einer Temperatur von 10 bis 100°C
durchführt.

Das molare Verhältnis der anorganischen oder organischen Protonensäure zum tertiären sp³-hybridisierten Amin oder sp²-hybridisierten Imin beträgt beim erfindungsgemäßen Verfahren im Allgemeinen 0,9 bis 1,5, bevorzugt 0,95 bis 1,1, besonders bevorzugt 0,95 bis 1,05 und ganz besonders bevorzugt 0,99 bis 1,02.

Bevorzugt setzt man beim erfindungsgemäßen Verfahren eine anorganische oder organische Protonensäure ein, deren teil- oder volldeprotoniertes Anion
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat, Vinylphosphonat, Dicyanamid, Bis(penta-fluoroethyl)phosphinat, Tris(pentafluoroethyl)trifluorophosphat, Tris(hepta-fluoro-propyl)trifluorophosphat, Bis[oxalato(2-)]borat, Bis[salicylato(2-)]borat, Bis[1,2-benzol-diolato(2-)-O,O']borat, Tetracyanoborat, Tetracarbonylcobaltat;
tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(CyF_{2y+1-z}H_{z})₆₋ₓ]⁻, wobei 1 ≤ x ≤ 6, 1 ≤ y ≤ 8 und 0 ≤ z ≤ 2y+1;
Imid der allgemeinen Formeln (Ve) [R⁹-SO₂-N-SO₂-R^{h}]⁻, (Vf) [Rⁱ-SO₂-N-CO-R^{j}]⁻ oder (IVg) [R^{k}-CO-N-CO-R^{l}]⁻, wobei R⁹ bis R^{l} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
Methid der allgemeinen Formel (Vh) 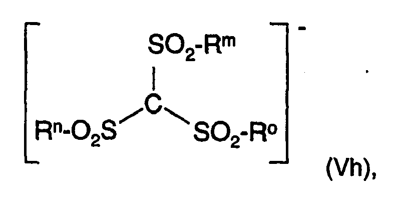 wobei R^{m} bis R^{o} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfat der allgemeinen Formel (Vi) [R^{p}O-SO₃]⁻, wobei R^{p} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Halometallat der allgemeinen Formel (Vj) [M_{q}Halᵣ]⁸⁻, wobei M für ein Metall und Hal für Fluor, Chlor, Brom oder lod steht, q und r ganze positive Zahlen sind und die Stöchiometrie des Komplexes angeben und s eine ganze positive Zahl ist und die Ladung des Komplexes angibt; oder
Sulfid, Hydrogensulfid, Hydrogenpolysulfid der allgemeinen Formel (Vk) [HSᵥ]⁻, Polysulfid der allgemeinen Formel (Vm) [Sᵥ]²⁻, wobei v eine ganze positive Zahl von 2 bis 10 ist, Thiolat der allgemeinen Formel (Vn) [R^{s}S]⁻, wobei R^{s} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
ist.

Als Heteroatome kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroa 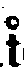 ome, so sind Sauerstoff, Stickstoff, Schwefel, Phosphor und Silizium bevorzugt. Als bevorzugte Gruppen seien insbesondere -O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂ (Amino), =NH (Imino), -COOH (Carboxy), -CONH₂ (Carboxamid) und -CN (Cyano) genannt. Fuktionelle Gruppen und Heteroatome können auch direkt benachbart sein, so dass auch Kombinationen aus mehreren benachbarten Atomen, wie etwa -O- (Ether), -S-(Thioether), -COO- (Ester), -CONH- (sekundäres Amid) oder -CONR- (tertiäres Amid), mit umfasst sind,

Als Halogene seien Fluor, Chlor, Brom und lod genannt.

Als Kohlenstoff enthaltende organische, gesättigte oder ungesättigte, acyclische oder cyclische, aliphatische, aromatische oder araliphatische Reste mit 1 bis 30 Kohlenstoffatomen stehen die Reste R^{a} bis R^{d} beim tetrasubstituiertes Borat (Va), der Rest R^{e} beim organischen Sulfonat (Vb), der Rest R^{f} beim Carboxylat (Vc), die Reste R^{g} bis R^{l} bei den Imiden (Ve), (Vf) und (Vg), die Reste R^{m} bis R^{o} beim Methid (Vh), der Rest R^{p} beim organischen Sulfat (Vi) und der Rest R^{s} beim Thiolat (Vn) unabhängig voneinander bevorzugt für
• C₁- bis C₃₀-Alkyl und deren aryl-, heteroaryl-, cycloalkyl-, halogen-, hydroxy-, amino-, carboxy-, formyl-, -O-, -CO-, -CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Formyl, Acetyl oder CₙF₂(ₙ₋ₐ)₊(_{1-b})H_{2a+b} mit n ≤ 30, 0 ≤ a ≤ n und b = 0 oder 1 (beispielsweise CF₃, C₂F₅, CH₂CH₂-C(ₙ-₂)F₂(ₙ₋₂)₊₁, C₆F₁₃, C₈F₁₇, C₁₀F₂₁, C₁₂F₂₅);
• C₃- bis C₁₂-Cycloalkyl und deren aryl-, heteroaryl-, cycloalkyl-, halogen-, hydroxy-, amino-, carboxy-, formyl-, -O-, -CO- oder -CO-O-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder CₙF_{2(n-a)-(1-b)}H_{2a-b} mit n ≤ 30, 0 ≤ a ≤ n und b = 0 oder 1;
• C₂- bis C₃₀-Alkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 2-Propenyl, 3-Butenyl, cis-2-Butenyl, trans-2-Butenyl oder CₙF_{2(n-a)-(1-b)}H_{2a-b} mit n ≤ 30, 0 ≤ a ≤ n und b = 0 oder 1;
• C₃- bis C₁₂-Cycloalkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1; und
• Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO-oder -CO-O-substituierte Komponenten, wie beispielsweise Phenyl, 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤ a ≤ 5.

Handelt es sich beim Anion um ein tetrasubstituiertes Borat (Va) [BR^{a}R^{b}R^{c}R^{d}]⁻, so sind bei diesem bevorzugt alle vier Reste R^{a} bis R^{d} identisch, wobei diese bevorzugt für Fluor, Trifluormethyl, Pentafluorethyl, Phenyl, 3,5-Bis(trifluormethyl)phenyl stehen. Besonders bevorzugte tetrasubstituierte Borate (Va) sind Tetrafluoroborat, Tetraphenylborat und Tetra[3,5-bis(trifluormethyl)phenyl]borat.

Handelt es sich beim Anion um ein organisches Sulfonat (Vb) [R^{e}-SO₃]⁻, so steht der Rest R^{e} bevorzugt für Methyl, Trifluormethyl, Pentafluorethyl, p-Tolyl oder C₉F₁₉. Besonders bevorzugte organische Sulfonate (Vb) sind Trifluormethansulfonat (Triflat), Methansulfonat, p-Tolylsulfonat, Nonadecafluorononansulfonat (Nonaflat), Dimethylenglykolmonomethyl-ethersulfat und Octylsulfat.

Handelt es sich beim Anion um ein Carboxylat (Vc) [R^{f}-COO]⁻, so steht der Rest R^{f} bevorzugt für Wasserstoff, Trifluormethyl, Pentafluorethyl, Phenyl, Hydroxy-phenylmethyl, Trichlormethyl, Dichlormethyl, Chlormethyl, Trifluormethyl, Difluormethyl, Fluormethyl, Ethenyl (Vinyl), 2-Propenyl, -CH=CH-COO⁻, cis-8-Heptadecenyl, -CH₂-C(OH)(COOH)-CH₂-COO⁻ oder unverzweigtes oder verzweigtes C₁- bis C₁₈-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Heptadecyl. Besonders bevorzugte Carboxylate (Vc) sind Formiat, Acetat, Propionat, Butyrat, Valeriat, Benzoat, Mandelat, Trichloracetat, Dichloracetat, Chloracetat, Trifluoracetat, Difluoracetat, Fluoracetat.

Handelt es sich beim Anion um ein (Fluoralkyl)fluorphosphat (Vd) [PFₓ(C_{y}F_{2y+1-z}H_{z})₆₋ₓ]⁻, so ist z bevorzugt 0. Besonders bevorzugt sind (Fluoralkyl)fluorphosphate (Vd), bei denen z = 0, x = 3 und 1 ≤ y ≤ 4, konkret [PF₃(CF₃)₃]⁻, [PF₃(C₂F₅)₃]⁻, [PF₃(C₃F₇)₃]⁻ und [PF₃(C₄F₇)₃]⁻.

Handelt es sich beim Anion um ein Imid (Ve) [R^{g}-SO₂-N-SO₂-R^{h}]⁻, (Vf) [R^{l}-SO₂-N-CO-R^{J}]⁻ oder (Vg) [R^{k}-CO-N-CO-R^{l}]⁻, so stehen die Reste R^{g} bis R^{l} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Imide (Ve), (Vf) und (Vg) sind [F₃C-SO₂-N-SO₂-CF₃]⁻ (Bis(trifluoromethylsulfonyl)imid), [F₅C₂-SO₂-N-SO₂-C₂F₅]⁻ (Bis(pentafluoroethylsulfonyl)imid), [F₃C-SO₂-N-CO-CF₃]⁻, [F₃C-CO-N-CO-CF₃]⁻ und jene, in denen die Reste R^{g} bis R^{l} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

### Handelt es sich beim Anion um ein Methid (Vh)

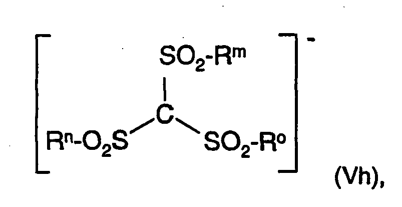

so stehen die Reste R^{m} bis R^{o} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Methide (Vh) sind [(F₃C-SO₂)₃C]⁻ (Tris(trifluoromethylsulfonyl)methid), [(F₅C₂-SO₂)₃C]⁻ (Bis(pentafluoroethylsulfonyl)-methid) und jene, in denen die Reste R^{m} bis R^{o} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

Handelt es sich beim Anion um ein organisches Sulfat (Vi) [R^{p}O-SO₃]⁻, so steht der Rest R^{p} bevorzugt für einen verzweigten oder unverzweigten C₁- bis C₃₀-Alklylrest. Besonders bevorzugte organische Sulfate (Vi) sind Methylsulfat, Ethylsulfat, Propylsulfat, Butylsulfat, Pentylsulfat, Hexylsulfat, Heptylsulfat oder Octylsulfat.

Handelt es sich beim Anion um ein Halometallat (Vj) [MqHalᵣ]^{s-}, so steht M bevorzugt für Aluminium, Zink, Eisen, Cobald, Antimon oder Zinn. Hal steht bevorzugt für Chlor oder Brom und ganz besonders bevorzugt für Chlor. q ist bevorzugt 1, 2 oder 3 und r und s ergeben sich entsprechend der Stöchiometrie und Ladung des Metallions.

Handelt es sich beim Anion um Thiolat (Vn) [R^{s}S]⁻, so steht der Rest R^{s} bevorzugt für einen verzweigten oder unverzweigten C₁- bis C₃₀-Alklylrest. Besonders bevorzugte Thiolate (Vn) sind Methylsulfid, Ethylsulfid, n-Propylsulfid, n-Butylsulfid, n-Pentylsulfid, n-Hexylsulfid, n-Heptylsulfid, n-Octylsulfid oder n-Dodecylsulfid.

Ganz besonders bevorzugt stellt man beim erfindungsgemäßen Verfahren als quartäre Ammoniumverbindung ein quartäres Ammoniumsalz her, bei dem das teil- oder volldeprotonierte Anion Tetrafluoroborat, Hexafluorophosphat, Trifluormethansulfonat, Methansulfonat, Formiat, Acetat, Mandelat, Nitrat, Nitrit, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇⁻, Chlorozinkat, Chloroferrat, Bis(trifluoromethylsulfonyl)imid, Bis(pentafluoroethylsulfonyl)imid, Tris(trifluoromethylsulfonyl)methid, Bis(pentafluoro-ethylsulfonyl)methid, p-Tolylsulfonat, Bis[salicylato(2-)]borat, Tetracarbonylcobaltat, Dimethylenglykolmonomethylethersulfat, Octylsulfat, Oleat, Stearat, Acrylat, Methacrylat, Maleinat, Hydrogencitrat, Vinylphosphonat, Bis(pentafluoroethyl)phosphinat, Bis[oxalato(2-)]borat, Bis[1,2-benzoldiolato(2-)-O,O']borat, Dicyanamid, Tris(penta-fluoroethyl)trifluorophosphat, Tris(heptafluoropropyl)trifluorophosphat, Tetracyanoborat oder Chlorocobaltat ist.

Der pKₐ-Wert der beim erfindungsgemäßen Verfahren einzusetzenden anorganischen oder organischen Protonensäure beträgt 1,8 bis 14, bevorzugt 1,8 bis 10, besonders bevorzugt 2 bis 10 und ganz besonders bevorzugt 3 bis 10, gemessen bei 25°C in wässriger Lösung.

Das molare Verhältnis von Dimethylsulfit zum tertiären sp³-hybridisierten Amin oder sp²-hybridisierten Imin beträgt beim erfindungsgemäßen Verfahren im Allgemeinen 0,9 bis 1,5, bevorzugt 0,9 bis 1,2, besonders bevorzugt 0,9 bis 1,1 und ganz besonders bevorzugt 0,95 bis 1,05 und insbesondere 0,99 bis 1,02.

Die Umsetzung zwischen dem tertiären sp³-hybridisierten Amin oder sp²-hybridisierten Imin, dem Dimethylsulfit und der anorganischen oder organischen Protonensäure erfolgt beim erfindungsgemäßen Verfahren bei einer Temperatur von 10 bis 100°C und einem Druck von 0,05 bis 2 MPa abs, bevorzugt 0,09 bis 0,5 MPa abs, besonders bevorzugt 0,09 bis 0,2 MPa abs und ganz besonders bevorzugt 0,095 bis 0,12 MPa abs.

Die für die Umsetzung erforderliche Zeitdauer ist in erster Linie abhängig von der chemischen Natur der Edukte (Reaktivität des tertiären sp³-hybridisierten Amins oder sp²-hybridisierten Imins und der anorganischen oder organischen Protonensäure) und der gewählten Umsetzungstemperatur. Sie kann etwa durch Vorversuche, in denen beispielsweise die Reaktionskinetik bestimmt wird, der Temperaturverlauf der exothermen Reaktion gemessen wird und/oder die Konzentrationen der Edukte und des Produkts analytisch bestimmt werden, ermittelt werden. Im Allgemeinen liegt die erforderliche Zeitdauer im Bereich wenigen Minuten bis zu einem Tag, in der Regel in der Größenordnung von 0,5 bis 24 Stunden, bevorzugt in der Größenordnung von 0,5 bis 10 Stunden.

Als Reaktionsapparate können beim erfindungsgemäßen Verfahren prinzipiell alle Reaktionsapparate eingesetzt werden, welche für eine Umsetzung in der Flüssigphase geeignet sind. Dies sind insbesondere Reaktionsapparate, welche eine entsprechende Vermischung der flüssigen Edukte ermöglichen, beispielsweise Rührkessel.

Die Art und Reihenfolge der Zugabe der einzelnen Edukte ist beim erfindungsgemäßen Verfahren unwesentlich. So ist es beispielsweise möglich, das tertiäre sp³-hybridisierte Amin oder sp²-hybridisierte Imin, das Dimethylsulfit und die anorganische oder organische Protonensäure nacheinander in beliebiger Reihenfolge oder parallel in den Reaktionsapparat zu geben. Es ist auch möglich, eines der drei Edukte vorzulegen und die anderen beiden Edukte über eine gewisse Zeitspanne von wenigen Minuten bis mehreren Stunden hinweg zuzutropfen.

Beim erfindungsgemäßen Verfahren ist es auch möglich, die Umsetzung in Gegenwart eines Lösungsmittels durchzuführen. Wird ein Lösungsmittel eingesetzt, so wählt man bevorzugt ein Lösungsmittel mit relativ geringer Polarität. Geeignete Lösungsmittel sind beispielsweise aromatische Kohlenwasserstoffe mit 6 bis 10 Kohlenstoffatomen, symmetrische oder unsymmetrische Dialkylether mit insgesamt 5 bis 10 Kohlenstoffatomen, Cycloalkane mit 5 bis 8 Kohlenstoffatomen oder C₅- bis C₁₀-Alkane. Als konkrete Beispiele seien Toluol, Xylol, Ethylbenzol, Diethylbenzol, Methyl-tert.-butylether, Cyclohexan, Hexan, Heptan und Oktan genannt. Bevorzugt führt man die erfindungsgemäße Umsetzung jedoch in Abwesenheit von Lösungsmitteln durch.

Bei der Umsetzung bildet sich Schwefeldioxid und Methanol, wobei in der Regel der Großteil des gebildeten Schwefeldioxids bereits bei der Umsetzung entweicht. Je nach Umsetzungstemperatur entweicht auch der Großteil des gebildeten Methanols bei der Umsetzung oder verbleibt im Reaktionsgemisch. Um das Reaktionsgemisch vom restlichen Schwefeldioxid und Methanol zu befreien, ist es im Allgemeinen vorteilhaft, das Reaktionsgemisch nach erfolgter Umsetzung unter Vakuum zu stellen und/oder auf eine Temperatur oberhalb des Siedepunktes von Methanol und unterhalb der Zersetzungstemperatur der quartären Ammoniumverbindung zu erhitzen. Liegt kein Vakuum an, so erhitzt man bevorzugt auf eine Temperatur von 80 bis 150°C. Die erforderlichen Bedingungen zur Befreiung vom restlichen Schwefeldioxid und Methanol können durch Vorversuche einfach ermittelt werden, wobei hierzu vorteilhafterweise die Restgehalte von Schwefeldioxid und Methanol sowie eventuell mögliche Zersetzungsprodukte der quartären Ammoniumverbindung zu analysieren oder kontrollieren sind.

Je nach gewünschter Reinheit der quartären Ammoniumverbindung kann es vorteilhaft sein, das erhaltene Produkt anschließend einem Reinigungsschritt zu unterziehen. Ist das Produkt bei der Arbeitstemperatur flüssig, so kann dieses mit einem geeigneten Lösungsmittel, in dem sich die quartäre Ammoniumverbindung nicht oder nur sehr geringfügig löst, ausgeschüttelt werden. Geeignete Lösungsmittel hierzu sind im Allgemeinen Lösungsmittel mit relativ geringer Polarität wie beispielsweise aromatische Kohlenwasserstoffe mit 6 bis 10 Kohlenstoffatomen, symmetrische oder unsymmetrische Dialkylether mit insgesamt 5 bis 10 Kohlenstoffatomen, Cycloalkane mit 5 bis 8 Kohlenstoffatomen oder C₅- bis C₁₀-Alkane sowie Ester wie beispielsweise Essigsäureethylester. Ist die quartäre Ammoniumverbindung bei der Arbeitstemperatur fest, so kann diese beispielsweise mit einem geeigneten Lösungsmittel, in dem sich die quartäre Ammoniumverbindung nicht oder nur sehr geringfügig löst, gewaschen werden. Geeignete Lösungsmittel hierzu sind beispielsweise ebenfalls die zuvor genannten. Ferner kann die feste quartäre Ammoniumverbindung auch in einem geeigneten Lösungsmittel umkristallisiert werden. Geeignete Lösungsmittel hierzu sind Lösungsmittel, in denen sich die quartäre Ammoniumverbindung löst, beispielsweise Alkohole, Acetonitril, Tetrahydrofuran oder Nitrobenzol.

Je nach weiterer Verwendung der gegebenenfalls gereinigten quartären Ammoniumverbindung ist es vorteilhaft, diese zuvor noch zu trocknen, beispielsweise im Vakuum.

Das erfindungsgemäße Verfahren kann diskontinuierlich, halbkontinuierlich oder kontinuierlich durchgeführt werden. Bei der diskontinuierlichen Durchführung gibt man die Edukte zusammen und führt die Umsetzung bei der gewünschten Temperatur durch. Nach Beendigung der Umsetzung wird das Reaktionsgemisch wie beschrieben aufgearbeitet. Bei der kontinuierlichen Durchführung gibt man alle drei Edukte zur Umsetzung bei der gewünschten Temperatur langsam in den Reaktionsapparat. Das Reaktionsgemisch wird dabei entsprechend der Mengen an zugeführten Edukten kontinuierlich entnommen und wie beschrieben aufgearbeitet. Die Aufarbeitung selbst kann dabei ebenfalls kontinuierlich erfolgen. Bei den halbkontinuierlichen Varianten gibt man mindestens eines, bevorzugt zwei der drei Edukte bei der gewünschten Temperatur langsam zu, wobei die Umsetzung im Allgemeinen parallel mit der Zugabe erfolgt. Nachdem die gewünschte(n) Menge(n) zugegeben wurde(n) beläßt man das Reaktionsgemisch im Allgemeinen noch für eine gewisse Zeit zur Nachreaktion und arbeitet anschließend wie beschrieben auf.

Beim erfindungsgemäßen Verfahren setzt man als tertiäres sp³-hybridisiertes Amin oder tertiäres sp²-hybridisiertes Imin bevorzugt ein Amin, ein Imidazol, ein Pyridin oder ein Guanidin ein.

Beim erfindungsgemäßen Verfahren setzt man als tertiäres sp³-hybridisiertes Amin bevorzugt ein Amin der allgemeinen Formel (I) 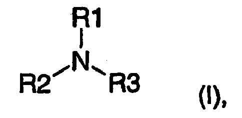 in der
die Reste R¹ bis R³ unabhängig voneinander einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten, wobei der Rest R¹ zusätzlich auch für Wasserstoff stehen kann; oder
der Rest R¹ wie zuvor definiert ist und die Reste R² und R³ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten; oder
die Reste R¹, R² und R³ zusammen einen dreibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 40 Kohlenstoffatomen bedeuten;
ein.

Beim erfindungsgemäßen Verfahren setzt man als tertiäres sp²-hybridisiertes Imin bevorzugt ein Imidazol der allgemeinen Formel (II) 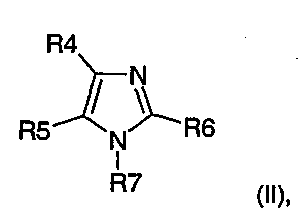 in der
die Reste R⁴ bis R⁷ unabhängig voneinander einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten und die Reste R⁴ bis R⁶ zusätzlich noch unabhängig voneinander Wasserstoff, Halogen oder eine funktionelle Gruppe bedeuten und der Rest R⁷ zusätzlich auch für Wasserstoff stehen kann; oder
zwei benachbarte Reste zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und der verbleibende Rest wie zuvor definiert ist;
ein.

Beim erfindungsgemäßen Verfahren setzt man als tertiäres sp²-hybridisiertes Imin bevorzugt ein Pyridin der allgemeinen Formel (III) 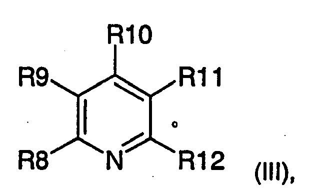 in der
die Reste R⁸ bis R¹² unabhängig voneinander Wasserstoff, Halogen, eine funktionelle Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten; oder
jeweils unabhängig voneinander zwei benachbarte Reste zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste/der verbleibende Rest wie zuvor definiert sind/ist;
ein.

Beim erfindungsgemäßen Verfahren setzt man als tertiäres sp²-hybridisiertes Imin bevorzugt ein Guanidin der allgemeinen Formel (IV) 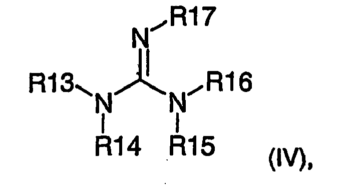 in der
die Reste R¹³ bis R¹⁷ unabhängig voneinander einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten, wobei die Reste R¹³ und R¹⁵ unabhängig voneinander zusätzlich auch für Wasserstoff stehen können; oder
jeweils unabhängig voneinander die Reste R¹³ und R¹⁴ und/oder R¹⁵ und R¹⁶ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste/der verbleibende Rest wie zuvor definiert sind/ist; oder
die Reste R¹⁴ und R¹⁵ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
ein.

Als Heteroatome kommen bei der Definition der Reste R¹ bis R¹⁷ prinzipiell alle Heteroatome in Frage, welche in der Lage sind, formell eine -CH₂-, eine -CH=, eine -C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt. Der Kohlenstoff enthaltende Rest kann dabei im Falle von R⁴ bis R⁶ und R⁸ bis R¹² auch direkt über das Heteroatom an den lmidazolium- bzw. Pyridinium-Ring gebunden sein.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂(Amino), =NH (Imino), -COOH (Carboxy), -CONH₂ (Carboxamid), -SO₃H (Sulfo) und -CN (Cyano) genannt. Fuktionelle Gruppen und Heteroatome können auch direkt benachbart sein, so dass auch Kombinationen aus mehreren benachbarten Atomen, wie etwa -O-(Ether), -S- (Thioether), -COO- (Ester), -CONH- (sekundäres Amid) oder -CONR- (tertiäres Amid), mit umfasst sind, beispielsweise Di-(C₁-C₄-Alkyl)-amino, C₁-C₄-Alkyloxycarbonyl oder C₁-C₄-Alkyloxy.

Als Halogene seien Fluor, Chlor, Brom und lod genannt.

Bevorzugt werden beim erfindungsgemäßen Verfahren Amine (I), Imidazole (II), Pyridine (III) und Guanidine (IV) eingesetzt, bei denen die Reste R⁴ bis R⁶ und R⁸ bis R¹² unabhängig voneinander
• Wasserstoff;
• Halogen; oder
• eine funktionelle Gruppe;
und die Reste R¹ bis R¹⁷ unabhängig voneinander jeweils
• gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₁- bis C₁₈-Alkyl;
• gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂- bis C₁₈-Alkenyl;
• gegebenenfalls durch funktionelle Gruppen, Aryl, Alkyl, Aryloxy, Alkyloxy, Halogen, Heteroatome und/oder Heterocyclen substituiertes C₆- bis C₁₂-Aryl;
• gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkyl;
• gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkenyl; oder
• einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus bedeuten; oder
benachbarte Reste R¹ und R², R² und R³, R¹ und R³, R⁴ und R⁵, R⁵ und R⁷, R⁷ und R⁶, R⁸ und R⁹, R⁹ und R¹⁰, R¹⁰ und R¹¹, R¹¹ und R¹², R¹³ und R¹⁴, R¹⁴ und R¹⁵, R¹⁵ und R¹⁶, R¹³ und R¹⁷ sowie R¹⁶ und R¹⁷ gemeinsam
• einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring bilden.

Bei gegebenenfalls durch funktionelle Gruppen, Aryl, Alkyl, Aryloxy, Alkyloxy, Halogen, Heteroatome und/oder Heterocyclen substituiertem C₁- bis C₁₈-Alkyl handelt es sich bevorzugt um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, 2-Etylhexyl, 2,4,4-Trimethylpentyl, 1,1,3,3-Tetramethylbutyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl, Cyclopentylmethyl, 2-Cyclopentylethyl, 3-Cyclopentylpropyl, Cyclohexylmethyl, 2-Cyclohexylethyl, 3-Cyclohexylpropyl, Benzyl (Phenylmethyl), Diphenylmethyl (Benzhydryl), Triphenylmethyl, 1-Phenylethyl, 2-Phenylethyl, 3-Phenylpropyl, α,α-Dimethylbenzyl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, Methoxy, Ethoxy, Formyl, 1,3-Dioxolan-2-yl, 1,3-Dioxan-2-yl, 2-Methyl-1,3-dioxolan-2-yl, 4-Methyl-1,3-dioxolan-2-yl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Acetyl, CₙF_{2(n-a)+(1-b)}H_{2a+b} mit n gleich 1 bis 30, 0 ≤ a ≤ n und b = 0 oder 1 (beispielsweise CF₃, C₂F₅, CH₂CH₂-C₍ₙ₋₂₎F₂₍ₙ₋₂₎₊₁, C₆F₁₃, C₈F₁₇, C₁₀F₂₁, C₁₂F₂₅), Chlormethyl, 2-Chlorethyl, Trichlormethyl, 1,1-Dimethyl-2-chlorethyl, Methoxymethyl, 2-Butoxyethyl, Diethoxymethyl, Diethoxyethyl, 2-lsopropoxyethyl, 2-Butoxypropyl, 2-Octyloxyethyl, 2-Methoxyisopropyl, 2-(Methoxycarbonyl)-ethyl, 2-(Ethoxycarbonyl)-ethyl, 2-(n-Butoxycarbonyl)-ethyl, Butylthiomethyl, 2-Dodecylthioethyl, 2-Phenylthioethyl, 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,1 0-dioxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dloxa-undecyl, 15-Methoxy-4,8,12-trioxa-pentadecyl, 9-Methoxy-5-oxa-nonyl, 14-Methoxy-5,10-dioxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bei gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂- bis C₁₈-Alkenyl handelt es sich bevorzugt um Vinyl, 2-Propenyl, 3-Butenyl, cis-2-Butenyl, trans-2-Butenyl oder CₙF_{2(n-a)-(1-b)}H_{2a-b} mit n ≤ 30, 0 ≤ a ≤ n und b = 0 oder 1.

Bei gegebenenfalls durch funktionelle Gruppen, Aryl, Alkyl, Aryloxy, Alkyloxy, Halogen, Heteroatome und/oder Heterocyclen substituiertes C₆- bis C₁₂-Aryl handelt es sich bevorzugt um Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2-Nitrophenyl, 4-Nitrophenyl, 2,4-Dinitrophenyl, 2,6-Dinitrophenyl, 4-Dimethylaminophenyl, 4-Acetylphenyl, Methoxyethylphenyl, Ethoxymethylphenyl, Methylthiophenyl, Isopropylthiophenyl oder tert.-Butylthiophenyl oder C₆F₍₅₋ₐ₎Hₐ mit 0 ≤ a ≤ 5.

Bei gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkyl handelt es sich bevorzugt um Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl, CₙF_{2(n-a)-(1-b)}H_{2a-b} mit n ≤ 30, 0 ≤ a ≤ n und b = 0 oder 1 sowie ein gesättigtes oder ungesättigtes bicyclisches System wie z.B. Norbornyl oder Norbornenyl.

Bei gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkenyl handelt es sich bevorzugt um 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1.

Bei einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus handelt es sich bevorzugt um Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl oder Difluorpyridyl.

Bilden die benachbarte Reste R¹ und R², R² und R³, R¹ und R³, R⁴ und R⁵, R⁵ und R⁷, R⁷ und R⁶, R⁸ und R⁹, R⁹ und R¹⁰, R¹⁰ und R¹¹, R¹¹ und R¹², R¹³ und R¹⁴, R¹⁴ und R¹⁵, R¹⁵ und R¹⁶, R¹³ und R¹⁷ sowie R¹⁶ und R¹⁷ gemeinsam einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring, so handelt es sich bevorzugt um 1,3-Propylen, 1,4-Butylen, 1,5-Pentylen, 2-Oxa-1,3-propylen, 1-Oxa-1,3-propylen, 2-Oxa-1,3-propylen, 1-Oxa-1,3-propenylen, 3-Oxa-1,5-pentylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Enthalten die oben genannten Reste Sauerstoff- und/oder Schwefelatome und/oder substituierte oder unsubstituierte Iminogruppen, so ist die Anzahl der Sauerstoff-und/oder Schwefelatome und/oder Iminogruppen nicht beschränkt. In der Regel beträgt sie nicht mehr als 5 in dem Rest, bevorzugt nicht mehr als 4 und ganz besonders bevorzugt nicht mehr als 3.

Enthalten die oben genannten Reste Heteroatome, so befinden sich zwischen zwei Heteroatomen in der Regel mindestens ein Kohlenstoffatom, bevorzugt mindestens zwei Kohlenstoffatome.

Besonders bevorzugt stehen die Reste R¹ bis R³, R⁷ sowie R¹³ bis R¹⁷ unabhängig voneinander für unverzweigtes oder verzweigtes C₁- bis C₁₂-Alkyl, wie beispieisweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl, 1-Octadecyl, 2-Hydroxyethyl, Benzyl, 3-Phenylpropyl, Vinyl, 2-Cyanoethyl, 2-(Methoxycarbonyl)-ethyl, 2-(Ethoxycarbonyl)-ethyl, 2-(n-Butoxy-carbonyl)-ethyl, Dimethylamino, Diethylamino, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl, für 6-Hydroxyhexyl oder Propylsulfonsäure. Zudem steht der Rest R⁷ besonders bevorzugt auch für eine Sulfo-Gruppe oder einen unverzweigten oder verzweigten Sulfo-C₁- bis C₁₂-alkyl-Rest.

Besonders bevorzugt stehen die Reste R⁴ bis R⁶ und R⁸ bis R¹² unabhängig voneinander für Wasserstoff oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino, Chlor, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl oder für 6-Hydroxyhexyl.

Ganz besonders bevorzugt setzt man beim erfindungsgemäßen Verfahren als Amin (I) Trimethylamin, Dimethylethylamin, Dimethyl-n-propylamin, Diethylmethylamin, Triethyl-amin, Tri-n-propylamin, Di-n-propylmethylamin, Tri-n-butylamin, Di-n-butylmethylamin, Tri-n-pentylamin, N-Methylpiperidin, N,N-Dimethylanilin und N-Methylmorpholin ein.

Ganz besonders bevorzugt setzt man beim erfindungsgemäßen Verfahren als Imidazol (II) N-Methylimidazol, N-Ethylimidazol, N-(1-Propyl)imidazol, N-(1-Butyl)imidazol, N-(1-Hexyl)imidazol, N-(1-Octyl)imidazol, N-(1-Decyl)imidazol, N-(1-Dodecyl)imidazol und N-(1-Pentadecyl)imidazol ein.

Ganz besonders bevorzugt setzt man beim erfindungsgemäßen Verfahren als Pyridin (III) Pyridin, 2-Methylpyridin, 3-Methylpyridin, 4-Methylpyridin, 2,4-Dimethylpyridin, 2,6-Dimethylpyridin, 2-Ethylpyridin und 2,6-Diethylpyridin ein.

Ganz besonders bevorzugt setzt man beim erfindungsgemäßen Verfahren als Guanidin (IV) N,N,N',N',N"-Pentamethylguanidin ein.

Werden beim erfindungsgemäßen Verfahren Amine eingesetzt, erfolgt die Umsetzung zwischen diesen, dem Dimethylsulfit und der anorganischen oder organischen Protonensäure bevorzugt bei einer Temperatur von 10 bis 80°C, besonders bevorzugt von 10 bis 60°C und ganz besonders bevorzugt von 10 bis 40°C.

Werden beim erfindungsgemäßen Verfahren Imidazole, Pyridine oder Guanidine eingesetzt, erfolgt die Umsetzung zwischen diesen, dem Dimethylsulfit und der anorganischen oder organischen Protonensäure bevorzugt bei einer Temperatur von 20 bis 100°C, besonders bevorzugt von 30 bis 90°C und ganz besonders bevorzugt von 50 bis 80°C.

In einer allgemeinen Ausführungsform legt man eines der drei Edukte vor und führt die anderen beiden Edukte parallel unter Vermischung über einen Zeitraum von wenigen Minuten bis mehreren Stunden bei der gewünschten Temperatur und dem gewünschten Druck zu. Nach beendeter Zugabe beläßt man im Allgemeinen das Reaktionsgemisch für weitere Minuten bis mehrere Stunden unter weiterem Rühren, wobei man vorteilhafterweise zur Abtrennung des restlichen Schwefeldioxids und Methanols ein Vakuum anlegt und/oder die Temperatur auf bis zu 150°C erhöht. Die erhaltene quartäre Ammoniumverbindung wird bevorzugt mit einem geeigneten Lösungsmittel gewaschen und anschließend im Vakuum getrocknet.

Das erfindungsgemäße Verfahren ermöglicht die Herstellung quartärer Ammoniumverbindungen mit flexibel wählbarem Anion in hoher Reinheit ohne aufwändige Reinigungsschritte, ist einfach durchzuführen und benötigt durch den Einsatz von Dimethylsulfit als Methylierungsmittel keine toxischen Substanzen. Trotz des Einsatzes von Dimethylsulfit wird beim erfindungsgemäßen Verfahren die Umlagerung des gebildeten Methylsulfits zum Methansulfonat fast vollständig vermieden oder zumindest deutlich zurückgedrängt, was maßgeblich die hohe Reinheit der quartären Ammoniumverbindungen auch bezüglich des möglichen Nebenprodukt-Anions Methansulfonat ermöglicht. Im Gegensatz dazu enthalten die nach dem Stand der Technik hergestellten quartären Ammoniumverbindungen signifikante Mengen an Methansulfonat-Anion, welches sich durch Umlagerung des Methylsulfit-Anions gebildet hat und sich nicht mehr in leicht flüchtige Komponenten zersetzen lässt.

Die nach dem erfindungsgemäßen Verfahren herstellbaren quartären Ammoniumverbindungen sind somit auch problemlos für den Einsatz in der Elektronikindustrie geeignet.

### Beispiele

### Beispiel 1 (erfindungsgemäß)

21,11 g (0,192 Mol) Dimethylsulfit wurden bei Raumtemperatur vorgelegt und unter Rühren innerhalb von 25 Minuten 23,8 g (0,192 Mol) N-Butylimidazol und 12 g (0,2 Mol) Essigsäure zugetropft. Das Reaktionsgemisch wurde 15 Stunden bei 60°C gerührt und anschließend 4 Stunden auf 120°C erhitzt und restliches Schwefeldioxid sowie Methanol abdestilliert. Das erhaltene Reaktionsprodukt wurde unter Vakuum 0,3 kPa (3 mbar) bei 100°C getrocknet. Die Ausbeute betrug 35,5 g, entsprechend 93% theoretischer Gesamtausbeute.

Das erhaltene flüssige Produkt wurde NMR-spektroskopisch analysiert und als N,N'-Butyl-methylimidazolium-acetat identifiziert:
[1 H-NMR, 400Mhz], D₂O.: 0,9 ppm (t - 3H); 1,3 ppm (m - 2H); 1,8 ppm (m - 2H); 1,9 ppm (s - 3H CH₃COO⁻); 2,8 ppm (s - 3H - CH₃SO3⁻); 3,4 ppm (s - 3H); 3,8 ppm (s - 3H); 4,2 ppm (t - 2H); 7,4 ppm (d - 2H); 8,7 ppm (s - 1H)

Eine quantitative Auswertung des NMR-Spektrums ergab aus dem Signalverhältnis 2,8 ppm (3H - Methansulfonat) : 3,8 ppm (3H - Methylgruppe am Imidazoliumstickstoff), dass der Anteil an gebildetem Methansulfonat unterhalb der Nachweisgrenze lag. Diese beträgt 3 Mol-%. Die Reinheit des N,N'-Butyl-methylimidazolium-acetats betrug somit > 97%.

### Beispiel 2 (Vergleichsbeispiel)

62 g (0,5 Mol) N-Butylimidazol wurde mit 55 g (0,5 Mol) Dimethylsulfit in einem 250 mL Vierhalskolben bei Raumtemperatur gemischt und auf 80°C erwärmt. Das Reaktionsgemisch wurde 5 Stunden lang gerührt und danach abgekühlt. Der Austrag wurde zweimal mit Essigsäureethylester ausgeschüttelt und anschließend unter Vakuum getrocknet. Die erhaltene Ausbeute betrug 108,6 g, entsprechend 92,8% theoretischer Gesamtausbeute (N,N'-Butyl-methylimidazolium-methylsulfit und -methansulfonat).

Zu 50,3 g (0,21 Mol) dieses Austrags wurde nun 13 g (0,21 Mol) Essigsäure gegeben und das Reaktionsgemisch auf 110°C erwärmt. Dabei wurde leichter Rückfluss beobachtet. Die flüchtigen Komponenten (Methanol und Schwefeldioxid) wurden unter Vakuum entfernt. Anschließend wurde der Austrag bei 140°C unter Vakuum getrocknet. Die erhaltene Ausbeute betrug 37,6 g, entsprechend 90% theoretischer Aubeute in Bezug auf das eingesetzte N,N'-Butyl-methylimidazolium-methylsulfit und -methansulfonat.

Die rechnerische Gesamtausbeute in Bezug auf das eingesetzte N-Butylimidazol betrug 92,8% 90% = 83,5%.

Das erhaltene flüssige Produkt wurde NMR-spektroskopisch analysiert und als Gemisch aus N,N'-Butyl-methylimidazolium-acetat und N,N'-Butyl-methylimidazolium-methansulfonat identifiziert:
[1 H-NMR, 400Mhz], D₂O.: 0,9 ppm (t - 3H); 1,3 ppm (m - 2H); 1,8 ppm (m - 2H); 1,9 ppm (s - 3H CH₃COO⁻); 2,8 ppm (s - 3H - CH₃SO₃⁻); 3,4 ppm (s - 3H); 3,8 ppm (s - 3H); 4,2 ppm (t - 2H); 7,4 ppm (d - 2H); 8,7 ppm (s - 1H)

Eine quantitative Auswertung des NMR-Spektrums ergab aus dem Signalverhältnis 2,8 ppm (3H - Methansulfonat) : 3,8 ppm (3H - Methylgruppe am Imidazoliumstickstoff), dass der Anteil an gebildetem Methansulfonat 21 Mol-% betrug. Die Reinheit des N,N'-Butyl-methylimidazolium-acetat betrug somit lediglich 79%.

Ein Vergleich der Beispiele 1 und 2 zeigt, dass durch das erfindungsgemäße Verfahren N,N'-Butyl-methylimidazolium-acetat in einer Reinheit von >97% und einer Gesamtausbeute von 93% erhalten werden kann, wohingegen die zweistufige Synthese nach Beispiel 2 lediglich eine Reinheit von 79% und eine Gesamtausbeute von 83,5% ermöglichte.

### Beispiel 3 (Vergleichsbeispiel mit Acetonitril als Lösungsmittel)

Beispiel 3 wurde im Wesentlichen analog Beispiel 1 von JP 2001-322,970 durchgeführt.

20,0 g (0,198 Mol) Triethylamin, 21,8 g (0,198 Mol) Dimethylsulfit und 40 mL Acetonitril wurden zusammengegeben und unter Atmosphärendruck und unter Rühren 2 Stunden unter Rückfluss erhitzt. Anschließend wurde das Acetonitril unter verminderten Druck abdestilliert und das flüssige Triethylmethylammonium-Salz erhalten. Dieses wurde in 100 mL Wasser gelöst und mit 38,0 g 50%-iger wässriger Tetrafluorborsäure, entsprechend 0,198 Mol HBF₄, versetzt. Die Lösung wurde auf 70°C erhitzt, wobei gebildetes Schwefeldioxid entwich. Nach Beendigung der Schwefeldioxid-Entwicklung wurde Wasser und Methanol unter Vakuum abdestilliert. Die theoretische Gesamtausbeute betrug 92% (Triethylmethylammonium-methylsulfit und -methansulfonat).

Gegenüber Beispiel 1 von JP 2001-322,970, worin eine Ausbeute von 96% beschrieben ist, betrug die Ausbeute in der Versuchsnachstellung nur 92%.

Das erhaltene flüssige Produkt wurde NMR-spektroskopisch analysiert und als Gemisch von Triethylmethylammonium-methylsulfit und Triethylmethylammoniummethansulfonat identifiziert:
[1H-NMR, 400Mhz], D₂O.: 1,3 ppm (t - 9H ); 2,8 ppm (s - 3H - Methansulfonat); 2,9 ppm (s - 3H); 3,3 ppm (q - 6H)

Zudem wurde über eine quantitative Auswertung des NMR-Spektrums aus dem Signalverhältnis 2,8 ppm (3H - Methansulfonat) : 2,9 ppm (3H - Methylgruppe am Ammonium-stickstoff) der Anteil an gebildetem Methansulfonat zu 6,1 Mol-% ermittelt. Die Reinheit des Triethylmethylammonium-methylsulfits betrug somit lediglich 93,9%.

### Beispiel 4 (Vergleichsbeispiel mit Acetonitril als Lösungsmittel)

Beispiel 4 wurde im Wesentlichen analog Beispiel 1 von JP 2001-322,970 durchgeführt, wobei anstelle von Triethylamin Pyridin eingesetzt wurde.

15,66 g (0,198 Mol) Pyridin, 21,8 g (0.198 Mol) Dimethylsulfit und 40 mL Acetonitril wurden zusammengegeben und unter Atmosphärendruck und unter Rühren 2 Stunden unter Rückfluss erhitzt. Anschließend wurde das Acetonitril unter verminderten Druck abdestilliert und das flüssige Methylpyridinium-Salz erhalten. Dieses wurde in 100 mL Wasser gelöst und mit 38,0 g 50%-iger wässriger Tetrafluorborsäure, entsprechend 0,198 Mol HBF₄, versetzt. Die Lösung wurde auf 70°C erhitzt, wobei gebildetes Schwefefdioxid entwich. Nach Beendigung der Schwefeldioxid-Entwicklung wurde Wasser und Methanol unter Vakuum abdestilliert. Die theoretische Gesamtausbeute betrug 86,8% (Methylpyridinium-methylsulfit und -methansulfonat).

Das erhaltene flüssige Produkt wurde NMR-spektroskopisch analysiert und als Gemisch von Methylpyridinium-methylsulfit und Methylpyridinium-methansulfonat identifiziert:
[1 H-NMR, 400Mhz], D₂O.: 2,8 ppm (s - 3H - Methansulfonat); 4,4 ppm (s - 3H); 4,45 ppm (s - 3H - Nebenkomponenten); 8,0 ppm (m, 2H); 8,5 ppm (m - 1H); 8,8 ppm (m - 2H)

Zudem wurde über eine quantitative Auswertung des NMR-Spektrums aus dem Signalverhältnis 2,8 ppm (3H - Methansulfonat) : 4,4 ppm (3H - Methylgruppe am Pyridinium-stickstoff) der Anteil an gebildetem Methansulfonat zu 10,5 Mol-% ermittelt. Die Reinheit des Pyridinium-methylsulfits betrug somit lediglich 89,5%.

## Patentansprüche

1. Verfahren zur Herstellung quartärer Ammoniumverbindungen durch Umsetzung des entsprechenden tertiären sp³-hybridisierten Amins oder sp²-hybridisierten Imins mit Dimethylsulfit, **dadurch gekennzeichnet, dass** man die Umsetzung
(i) in Gegenwart einer anorganischen oder organischen Protonensäure mit einem pKₐ-Wert von 1,8 bis 14, gemessen bei 25°C in wässriger Lösung; und
(ii) bei einer Temperatur von 10 bis 100°C
durchführt.

2. Verfahren nach Anspruch 1, **dadurch gekennzeichnet, dass** man ein molares Verhältnis der anorganischen oder organischen Protonensäure zum tertiären sp³-hybridisierten Amin oder sp²-hybridisierten Imin von 0,9 bis 1,5 einsetzt.

3. Verfahren nach den Ansprüchen 1 bis 2, **dadurch gekennzeichnet, dass** man als anorganischen oder organischen Protonensäure eine Säure einsetzt, deren teil-oder volldeprotoniertes Anion
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat, Vinylphosphonat, Dicyanamid, Bis(pentafluoroethyl)phosphinat, Tris(pentafluoroethyl)trifluorophosphat, Tris(heptafluoropropyl)trifluorophosphat, Bis[oxalato(2-)]borat, Bis[salicylato(2-)]borat, Bis[1,2-benzoldiolato(2-)-O,O']borat, Tetracyanoborat, Tetracarbonylcobaltat;
tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(C_{y}F_{2y+1-z}H_{z})₆₋ₓ]⁻, wobei 1 ≤ x ≤ 6, 1 ≤ y ≤ 8 und 0 ≤ z ≤ 2y+1;
Imid der allgemeinen Formeln (Ve) [R^{g}-SO₂-N-SO₂-R^{h}]⁻, (Vf) [R^{l}-SO₂-N-CO-R^{j}]⁻ oder (IVg) [R^{k}-CO-N-CO-R^{l}]⁻, wobei R^{g} bis R^{l} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
Methid der allgemeinen Formel (Vh) 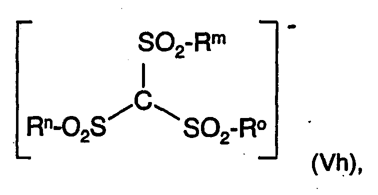 wobei R^{m} bis R^{o} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfat der allgemeinen Formel (Vi) [R^{p}O-SO₃]⁻, wobei R^{p} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Halometallat der allgemeinen Formel (Vj) [M_{q}Halᵣ]^{s-}, wobei M für ein Metall und Hal für Fluor, Chlor, Brom oder lod steht, q und r ganze positive Zahlen sind und die Stöchiometrie des Komplexes angeben und s eine ganze positive Zahl ist und die Ladung des Komplexes angibt; oder
Sulfid, Hydrogensulfid, Hydrogenpolysulfid der allgemeinen Formel (Vk) [HSᵥ]⁻, Polysulfid der allgemeinen Formel (Vm) [Sᵥ]²⁻, wobei v eine ganze positive Zahl von 2 bis 10 ist, Thiolat der allgemeinen Formel (Vn) [R^{s}S]⁻, wobei R^{s} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
ist.

4. Verfahren nach Anspruch 3, **dadurch gekennzeichnet, dass** man als anorganischen oder organischen Protonensäure eine Säure einsetzt, deren teil- oder volldeprotoniertes Anion Tetrafluoroborat, Hexafluorophosphat, Trifluormethansulfonat, Methansulfonat, Formiat, Acetat, Mandelat, Nitrat, Nitrit, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇⁻, Chlorozinkat, Chloroferrat, Bis(trifluoromethylsulfonyl)imid, Bis(pentafluoroethylsulfonyl)imid, Tris(trifluoromethylsulfonyl)methid, Bis(pentafluoroethylsulfonyl)methid, p-Tolylsulfonat, Bis[salicylato(2-)]borat, Tetracarbonylcobaltat, Dimethylenglykolmonomethylethersulfat, Octylsulfat, Oleat, Stearat, Acrylat, Methacrylat, Maleinat, Hydrogencitrat, Vinylphosphonat, Bis(pentafluoroethyl)phosphinat, Bis[oxalato(2-)]borat, Bis[1,2-benzoldiolato(2-)-O,O']borat, Dicyanamid, Tris(pentafluoroethyl)-trifluorophosphat, Tris(heptafluoropropyl)trifluorophosphat, Tetracyanoborat oder Chlorocobaltat ist.

5. Verfahren nach den Ansprüchen 1 bis 4, **dadurch gekennzeichnet, dass** man ein molares Verhältnis von Dimethylsulfit zum tertiären sp³-hybridisierten Amin oder sp²-hybridisierten Imin von 0,9 bis 1,5 einsetzt.

6. Verfahren nach den Ansprüchen 1 bis 4, **dadurch gekennzeichnet, dass** man als tertiäres sp³-hybridisiertes Amin ein Amin der allgemeinen Formel (I) 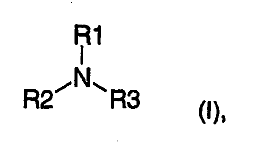 in der
die Reste R¹ bis R³ unabhängig voneinander einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten, wobei der Rest R¹ zusätzlich auch für Wasserstoff stehen kann; oder
der Rest R¹ wie zuvor definiert ist und die Reste R² und R³ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten; oder
die Reste R¹, R² und R³ zusammen einen dreibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 40 Kohlenstoffatomen bedeuten;
einsetzt.

7. Verfahren nach den Ansprüchen 1 bis 4, **dadurch gekennzeichnet, dass** man als tertiäres sp²-hybridisiertes Imin ein Imidazol der allgemeinen Formel (II) 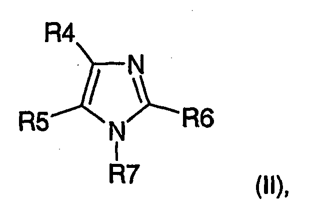 in der
die Reste R⁴ bis R⁷ unabhängig voneinander eine Sulfo-Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten und die Reste R⁴ bis R⁶ zusätzlich noch unabhängig voneinander Wasserstoff, Halogen oder eine funktionelle Gruppe bedeuten und der Rest R⁷ zusätzlich auch für Wasserstoff stehen kann; oder
zwei benachbarte Reste zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und der verbleibende Rest wie zuvor definiert ist;
einsetzt.

8. Verfahren nach den Ansprüchen 1 bis 4, **dadurch gekennzeichnet, dass** man als tertiäres sp²-hybridisiertes Imin ein Pyridin der allgemeinen Formel (III) 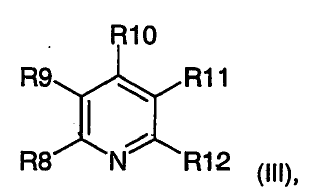 in der
die Reste R⁸ bis R¹² unabhängig voneinander Wasserstoff, Halogen, eine funktionelle Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten; oder
jeweils unabhängig voneinander zwei benachbarte Reste zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste/der verbleibende Rest wie zuvor definiert sind/ist;
einsetzt.

9. Verfahren nach den Ansprüchen 1 bis 4, **dadurch gekennzeichnet, dass** man als tertiäres sp²-hybridisiertes Imin ein Guanidin der allgemeinen Formel (IV) 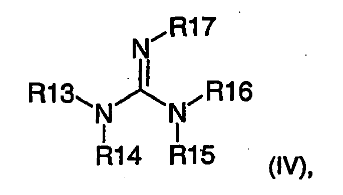 in der
die Reste R¹³ bis R¹⁷ unabhängig voneinander einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen bedeuten, wobei die Reste R¹³ und R¹⁵ unabhängig voneinander zusätzlich auch für Wasserstoff stehen können; oder
jeweils unabhängig voneinander die Reste R¹³ und R¹⁴ und/oder R¹⁵ und R¹⁶ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste/der verbleibende Rest wie zuvor definiert sind/ist; oder
die Reste R¹⁴ und R¹⁵ zusammen einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen bedeuten und die verbleibenden Reste wie zuvor definiert sind;
einsetzt.

10. Verfahren nach den Ansprüchen 1 bis 9, **dadurch gekennzeichnet, dass** man als tertiäres sp³-hybridisiertes Amin oder sp²-hybridisiertes Imin Trimethylamin, Dimethylethylamin, Dimethyl-n-propylamin, Diethylmethylamin, Triethyl-amin, Tri-n-propylamin, Di-n-propylmethylamin, Tri-n-butylamin, Di-n-butylmethylamin, Tri-n-pentylamin, N-Methylpiperidin, N,N-Dimethylanilin, N-Methylmorpholin, N-Methylimidazol, N-Ethylimidazol, N-(1-Propyl)imidazol, N-(1-Butyl)imidazol, N-(1-Hexyl)-imidazol, N-(1-Octyl)imidazol, N-(1-Decyl)imidazol, N-(1-Dodecyl)imidazol, N-(1-Pentadecyl)imidazol, Pyridin, 2-Methylpyridin, 3-Methylpyridin, 4-Methylpyridin, 2,4-Dimethylpyridin, 2,6-Dimethylpyridin, 2-Ethylpyridin, 2,6-Diethylpyridin oder N,N,N',N',N"-Pentamethylguanidin einsetzt.

## Claims

1. A process for preparing quaternary ammonium compounds by reacting the corresponding tertiary sp³-hybridized amine or sp²-hybridized imine with dimethyl sulfite, wherein the reaction is carried out
(i) in the presence of an inorganic or organic protic acid having a pKₐ of from 1.8 to 14, measured at 25°C in aqueous solution; and
(ii) at a temperature of from 10 to 100°C.

2. The process according to claim 1, wherein a molar ratio of the inorganic or organic protic acid to the tertiary sp³-hybridized amine or sp²-hybridized imine of from 0.9 to 1.5 is used.

3. The process according to claim 1 or 2, wherein the inorganic or organic protic acid used is an acid whose partially or fully deprotonated anion is
fluoride; hexafluorophosphate; hexafluoroarsenate; hexafluoroantimonate;
trifluoroarsenate; nitrite; nitrate; sulfate; hydrogensulfate; carbonate;
hydrogencarbonate; phosphate; hydrogenphosphate; dihydrogenphosphate,
vinylphosphonate, dicyanamide, bis(pentafluoroethyl)phosphinate,
tris(pentafluoroethyl)trifluorophosphate, tris(heptafluoropropyl)trifluorophosphate, bis[oxalato(2-)]borate, bis[salicylato(2-)]borate, bis[1,2-benzenediolato(2-)-O,O']borate, tetracyanoborate, tetracarbonylcobaltate;
tetrasubstituted borate of the general formula (Va) [BR^{a}R^{b}R^{c}R^{d}]⁻, where R^{a} to R^{d} are each, independently of one another, fluorine or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
organic sulfonate of the general formula (Vb) [R^{e}-SO₃]⁻, where R^{e} is a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
carboxylate of the general formula (Vc) [R^{f}-COO]⁻, where R^{f} is hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
(fluoroalkyl)fluorophosphate of the general formula (Vd) [PFₓ(C_{y}F_{2y+1-z}H_{z})₆₋ₓ]⁻,
where 1 ≤ x ≤ 6, 1 ≤ y ≤ 8 and 0 ≤ z ≤ 2y+1;
imide of the general formulae (Ve) [R^{g}-SO₂-N-SO₂-R^{h}]⁻ , (Vf) [Rⁱ-SO₂-N-CO-R^{j}]⁻ or (IVg) [R^{k}-CO-N-CO-R^{l}]⁻, where R^{g} to R¹ are each, independently of one another, hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
methide of the general formula (Vh) 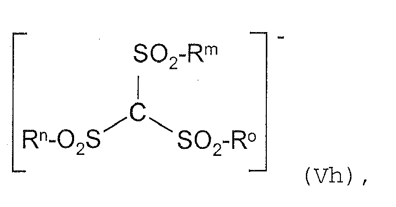 where R^{m} to R^{o} are, independently of one another, hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
organic sulfate of the general formula (Vi) [R^{p}O-SO₃]⁻, where R^{p} is a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen;
halometalate of the general formula (Vj) [M_{q}Halᵣ]^{s-}, where M is a metal and Hal is fluorine, chlorine, bromine or iodine, q and r are positive integers and indicate the stoichiometry of the complex and s is a positive integer and indicates the charge on the complex; or
sulfide, hydrogensulfide, hydrogenpolysulfide of the general formula (Vk) [HSᵥ]⁻, polysulfide of the general formula (Vm) [Sᵥ]²⁻, where v is a positive integer from 2 to 10, thiolate of the general formula (Vn) [R^{s}S]⁻, where R^{s} is a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogen.

4. The process according to claim 3, wherein the inorganic or organic protic acid used is an acid whose partially or fully deprotonated anion is tetrafluoroborate, hexafluorophosphate, trifluoromethanesulfonate, methanesulfonate, formate, acetate, mandelate, nitrate, nitrite, trifluoroacetate, sulfate, hydrogensulfate, methylsulfate, ethylsulfate, propylsulfate, butylsulfate, pentylsulfate, hexylsulfate, heptylsulfate, octylsulfate, phosphate, dihydrogenphosphate, hydrogenphosphate, propionate, tetrachloroaluminate, Al₂Cl₇⁻, chlorozincate, chloroferrate, bis(trifluoromethylsulfonyl)imide, bis(pentafluoroethylsulfonyl)imide, tris(trifluoromethylsulfonyl)methide, bis(pentafluoroethylsulfonyl)methide, p-toluenesulfonate, bis[salicylato(2-)]borate, tetracarbonylcobaltate, dimethylene glycol monomethyl ether sulfate, octylsulfate, oleate, stearate, acrylate, methacrylate, maleate, hydrogencitrate, vinylphosphonate, bis(pentafluoroethyl)phosphinate, bis[oxalato(2-)]borate, bis[1,2-benzenediolato(2-)-O,O']borate, dicyanamide, tris(pentafluoroethyl)trifluorophosphate, tris(heptafluoropropyl)trifluorophosphate, tetracyanoborate or chlorocobaltate.

5. The process according to any of claims 1 to 4, wherein a molar ratio of dimethyl sulfite to the tertiary sp³-hybridized amine or sp²-hybridized imine of from 0.9 to 1.5 is used.

6. The process according to any of claims 1 to 4, wherein an amine of the general formula (I) 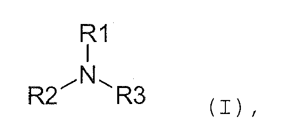 where
the radicals R¹ to R³ are each, independently of one another, a carbon-comprising organic, saturated or unsaturated, acyclic or cyclic, aliphatic, aromatic or araliphatic radical which has from 1 to 20 carbon atoms and is unsubstituted or interrupted or substituted by from 1 to 5 heteroatoms or functional groups, with the radical R¹ also being able to be hydrogen; or
the radical R¹ is as defined above and the radicals R² and R³ together form a divalent, carbon-comprising organic, saturated or unsaturated, acyclic or cyclic, aliphatic, aromatic or araliphatic radical which has from 1 to 30 carbon atoms and is unsubstituted or interrupted or substituted by from 1 to 5 heteroatoms or functional groups; or
the radicals R¹, R² and R³ together form a trivalent, carbon-comprising organic, saturated or unsaturated, acyclic or cyclic, aliphatic, aromatic or araliphatic radical which has from 1 to 40 carbon atoms and is unsubstituted or interrupted or substituted by from 1 to 5 heteroatoms or functional groups;
is used as tertiary sp³-hybridized amine.

7. The process according to any of claims 1 to 4, wherein an imidazole of the general formula (II) 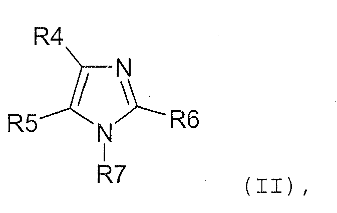 where
the radicals R⁴ to R⁷ are each, independently of one another, a sulfo group or a carbon-comprising organic, saturated or unsaturated, acyclic or cyclic, aliphatic, aromatic or araliphatic radical which has from 1 to 20 carbon atoms and is unsubstituted or interrupted or substituted by from 1 to 5 heteroatoms or functional groups and the radicals R⁴ to R⁶ may also be, independently of one another, hydrogen, halogen or a functional group and the radical R⁷ may also be hydrogen; or
two adjacent radicals together form a divalent, carbon-comprising organic, saturated or unsaturated, acyclic or cyclic, aliphatic, aromatic or araliphatic radical which has from 1 to 30 carbon atoms and is unsubstituted or interrupted or substituted by from 1 to 5 heteroatoms or functional groups and the remaining radical is as defined above;
is used as tertiary sp²-hybridized imine.

8. The process according to any of claims 1 to 4, wherein a pyridine of the general formula (III) 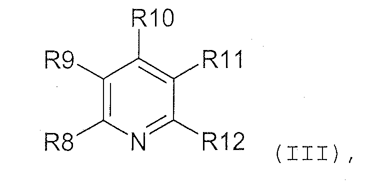 where
the radicals R⁸ to R¹² are each, independently of one another, hydrogen, halogen, a functional group or a carbon-comprising organic, saturated or unsaturated, acyclic or cyclic, aliphatic, aromatic or araliphatic radical which has from 1 to 20 carbon atoms and is unsubstituted or interrupted or substituted by from 1 to 5 heteroatoms or functional groups; or
in each case independently, two adjacent radicals together form a divalent, carbon-comprising organic, saturated or unsaturated, acyclic or cyclic, aliphatic, aromatic or araliphatic radical which has from 1 to 30 carbon atoms and is unsubstituted or interrupted or substituted by from 1 to 5 heteroatoms or functional groups and the remaining radicals/radical are/is as defined above;
is used as tertiary sp²-hybridized imine.

9. The process according to any of claims 1 to 4, wherein a guanidine of the general formula (IV) 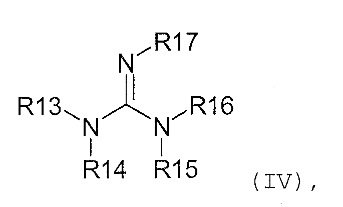 where
the radicals R¹³ to R¹⁷ are each, independently of one another, a carbon-comprising organic, saturated or unsaturated, acyclic or cyclic, aliphatic, aromatic or araliphatic radical which has from 1 to 20 carbon atoms and is unsubstituted or interrupted or substituted by from 1 to 5 heteroatoms or functional groups, with the radicals R¹³ and R¹⁵ also being able, independently of one another, to be hydrogen; or,
in each case independently, the radicals R¹³ and R¹⁴ and/or R¹⁵ and R¹⁶ together form a divalent, carbon-comprising organic, saturated or unsaturated, acyclic or cyclic, aliphatic, aromatic or araliphatic radical which has from 1 to 30 carbon atoms and is unsubstituted or interrupted or substituted by from 1 to 5 heteroatoms or functional groups and the remaining radicals/radical are/is as defined above; or
the radicals R¹⁴ and R¹⁵ together form a divalent, carbon-comprising organic, saturated or unsaturated, acyclic or cyclic, aliphatic, aromatic or araliphatic radical which has from 1 to 30 carbon atoms and is unsubstituted or interrupted or substituted by from 1 to 5 heteroatoms or functional groups and the remaining radicals are as defined above;
is used as tertiary sp²-hybridized imine.

10. The process according to any of claims 1 to 9, wherein the tertiary sp³-hybridized amine or sp²-hybridized imine used is trimethylamine, dimethylethylamine, dimethyl-n-propylamine, diethylmethylamine, triethylamine, tri-n-propylamine, di-n-propylmethylamine, tri-n-butylamine, di-n-butylmethylamine, tri-n-pentylamine, N-methylpiperidine, N,N-dimethylaniline, N-methylmorpholine, N-methylimidazole, N-ethylimidazole, N-(1-propyl)imidazole, N-(1-butyl)imidazole, N-(1-hexyl)imidazole, N-(1-octyl)imidazole, N-(1-decyl)imidazole, N-(1-dodecyl)imidazole, N-(1-pentadecyl)imidazole, pyridine, 2-methylpyridine, 3-methylpyridine, 4-methylpyridine, 2,4-dimethylpyridine, 2,6-dimethylpyridine, 2-ethylpyridine, 2,6-diethylpyridine or N,N,N',N',N"-pentamethylguanidine.

## Revendications

1. Procédé pour la préparation de composés d'ammonium quaternaire, par mise en réaction de l'amine tertiaire sp³-hybridée correspondante ou de l'imine tertiaire sp²-hybridée correspondante avec du sulfite de diméthyle, **caractérisé en ce qu'**on effectue la réaction
(i) en présence d'un acide protonique organique ou inorganique, ayant un pKₐ de 1,8 à 14, mesuré en solution aqueuse à 25°C ; et
(ii) à une température de 10 à 100 °C.

2. Procédé selon la revendication 1, **caractérisé en ce qu'**on utilise un rapport molaire de l'acide protonique organique ou inorganique à l'amine tertiaire sp³-hybridée ou à l'imine tertiaire sp²-hybridée de 0,9 à 1,5.

3. Procédé selon les revendications 1 et 2, **caractérisé en ce qu'**on utilise comme acide protonique organique ou inorganique un acide dont l'anion partiellement ou totalement protoné est l'anion
fluorure ; hexafluorophosphate ; hexafluoro-arsénate, hexafluoroantimonate ; trifluoro-arsénate ; nitrite ; nitrate ; sulfate ; hydrogénosulfate ; carbonate ; hydrogéno-carbonate ; phosphate ; hydrogénophosphate ; dihydrogénophosphate, vinylphosphonate, dicyanamide, bis(pentafluoroéthyl)phosphinate, tris(pentafluoroéthyl)trifluorophosphate, tris-(heptafluoropropyl)trifluorophosphate, bis-[oxalato(2-)]borate, bis[salicylato(2-)]borate, bis[1,2-benzènediolato(2-)O,O']borate, tétracyanoborate ; tétracarbonylcobaltate ;
borate tétrasubstitué de formule générale (Va) [BR^{a}R^{b}R^{c}R^{d}]⁻, dans laquelle R^{a} à R^{d} représentent, indépendamment l'un de l'autre, un atome de fluor ou un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
sulfonate organique de formule générale (Vb) [R^{e}-SO₃]⁻, dans laquelle R^{e} représente un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
carboxylate de formule générale (Vc) [R^{f}-COO]⁻, dans laquelle R^{f} représente un atome d'hydrogène ou un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
(fluoroalkyl)fluorophosphate de formule générale (Vd) [PFₓ(C_{y}F_{2y+2}H_{z})₆₋ₓ]⁻, où 1 ≤ x ≤ 6, 1 ≤ y ≤ 8 et 0 ≤ z ≤ 2y+1 ;
imide de formule générales (Ve) [R^{g}-SO₂-N-SO₂-R^{h}]⁻, (Vf) [R^{l}-SO₂-N-SO₂-R^{l}]⁻ ou (IVg) [R^{k}-CO-N-CO-R^{l}]⁻, dans lesquelles R^{g} à R^{l} représentent, indépendamment les uns des autres, un atome d'hydrogène ou un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
méthide de formule générale (Vh) 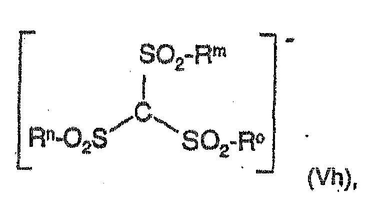 dans laquelle R^{m} à R^{o} représentent, chacun indépendamment, un atome d'hydrogène ou un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
sulfate organique de formule générale (Vi) [R^{p}O-SO₃]⁻, dans laquelle R^{p} représente un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels ;
halgénométallate de formule générale (Vj) [M_{q}Halᵣ]^{s-}, dans laquelle M représente un métal et Hal représente le fluor, le chlore, le brome ou l'iode, q et r sont des nombres entiers positifs et indiquent la stoechiométrie du complexe, et s est un nombre entier positif et indique la charge du complexe ; ou
sulfure, hydrogénosulfure, hydrogénopolysulfure de formule générale (Vk) [HSᵥ]⁻, polysulfure de formule générale (Vm) [Sᵥ]²⁻, où v est un nombre entier positif allant de 2 à 10, thiolate de formule générale (Vn) [P^{s}S]⁻, où R^{s} représente un radical organique contenant du carbone, saturé ou insaturé, cyclique ou acyclique, aliphatique, aromatique ou araliphatique, ayant de 1 à 30 atomes de carbone, qui peut contenir un ou plusieurs hétéroatomes et/ou être substitué par un ou plusieurs atomes d'halogène ou groupes fonctionnels.

4. Procédé selon la revendication 3, **caractérisé en ce qu'**on utilise en tant qu'acide protonique organique ou inorganique un acide dont l'anion partiellement ou totalement protoné est l'anion tétrafluoroborate, hexafluorophosphate, trifluoro-méthanesulfonate, méthanesulfonate, formiate, acétate, mandélate, nitrate, nitrite, trifluoro-acétate, sulfate, hydrogénosulfate, méthylsulfate, éthylsulfate, propylsulfate, butylsulfate, pentylsulfate, hexylsulfate, heptylsulfate, octylsulfate, phosphate, dihydrogénophosphate, hydrogénophosphate, propionate, tétrachloro-aluminate, Al₂Cl₇⁻, chlorozincate, chloroferrate, bis(trifluorométhylsulfonyl)imide, bis(penta-fluoroéthylsulfonyl)imide, tris(trifluorométhyl-sulfonyl)méthide, bis(pentafluoroéthylsulfonyl)-méthide, p-tolylsulfonate, bis[salicylato(2-)]-borate, tétracarbonylcobaltate, diméthylèneglycol-monométhyléthersulfate, octylsulfate, oléate, stéarate, acrylate, méthacrylate, maléate, hydrogénocitrate, vinylphosphonate, bis(penta-fluoroéthyl)phosphinate, bis[oxalato(2-)]borate, bis[1,2-benzènediolato(2-)-O,O']borate, dicyanamide, tris[pentafluoroéthyl)trifluoro-phosphate, tris(heptafluoropropyl)trifluoro-phosphate, tétracyanoborate ou chlorocobaltate.

5. Procédé selon les revendications 1 à 4, **caractérisé en ce qu'**on utilise un rapport molaire du sulfite de diméthyle à l'amine tertiaire sp³-hybridée ou l'imine tertiaire sp²-hybridée allant de 0,9 à 1,5.

6. Procédé selon les revendications 1 à 4, **caractérisé en ce qu'**on utilise en tant qu'amine tertiaire sp³-hybridée une amine de formule générale (I) 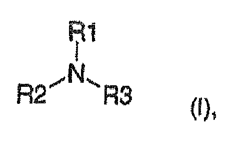 dans laquelle
les radicaux R¹ à R³ représentent, chacun indépendamment, un radical organique contenant du carbone, saturé ou insaturé, cyclique ou acyclique, aliphatique, aromatique ou araliphatique, ayant de 1 à 20 atomes de carbone, non substitué ou interrompu ou substitué par 1 à 5 hétéroatomes ou groupes fonctionnels, le radical R¹ pouvant en outre représenter également un atome d'hydrogène ; ou
le radical R¹ est tel que défini précédemment et les radicaux R² et R³ représentent ensemble un radical organique à deux liaisons, contenant du carbone, saturé ou insaturé, cyclique ou acyclique, aliphatique, aromatique ou araliphatique, ayant de 1 à 30 atomes de carbone, non substitué ou interrompu ou substitué par 1 à 5 hétéroatomes ou groupes fonctionnels ; ou
les radicaux R¹, R² et R³ représentent ensemble un radical organique à trois liaisons, contenant du carbone, saturé ou insaturé, cyclique ou acyclique, aliphatique, aromatique ou araliphatique, ayant de 1 à 40 atomes de carbone, non substitué ou interrompu ou substitué par 1 à 5 hétéroatomes ou groupes fonctionnels.

7. Procédé selon les revendications 1 à 4, **caractérisé en ce qu'**on utilise en tant qu'imine tertiaire sp²-hybridée un imidazole de formule générale (II) 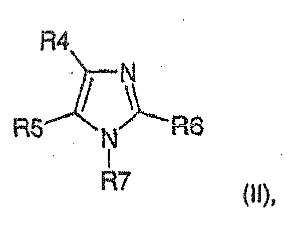 dans laquelle
les radicaux R⁴ à R⁷ représentent, indépendamment les uns des autres, un groupe sulfo ou un radical organique contenant du carbone, saturé ou insaturé, cyclique ou acyclique, aliphatique, aromatique ou araliphatique, ayant de 1 à 20 atomes de carbone, non substitué ou interrompu ou substitué par 1 à 5 hétéroatomes ou groupes fonctionnels, et les radicaux R⁴ à R⁶ en outre représentent encore, indépendamment les uns des autres, un atome d'hydrogène, un atome d'halogène ou un groupe fonctionnel, et le radical R⁷ peut en outre représenter également un atome d'hydrogène ; ou
deux radicaux contigus représentent ensemble un radical organique à deux liaisons, contenant du carbone, saturé ou insaturé, cyclique ou acyclique, aliphatique, aromatique ou araliphatique, ayant de 1 à 30 atomes de carbone, non substitué ou interrompu ou substitué par 1 à 5 hétéroatomes ou groupes fonctionnels, et le radical restant est tel que défini précédemment.

8. Procédé selon les revendications 1 à 4, **caractérisé en ce qu'**on utilise en tant qu'imine tertiaire sp²-hybridée une pyridine de formule générale (III) 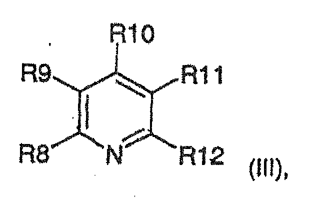 dans laquelle
les radicaux R⁸ à R¹² représentent, indépendamment les uns des autres, un atome d'hydrogène, un atome d'halogène, un groupe fonctionnel ou un radical organique contenant du carbone, saturé ou insaturé, cyclique ou acyclique, aliphatique, aromatique ou araliphatique, ayant de 1 à 20 atomes de carbone, non substitué ou interrompu ou substitué par 1 à 5 hétéroatomes ou groupes fonctionnels ; ou
chaque fois indépendamment les uns des autres, deux radicaux contigus représentent ensemble un radical organique a deux liaisons, contenant du carbone, saturé ou insaturé, cyclique ou acyclique, aliphatique, aromatique ou araliphatique, ayant de 1 à 30 atomes de carbone, non substitué ou interrompu ou substitué par 1 à 5 hétéroatomes ou groupes fonctionnels, et le radical restant est tel que défini précédemment.

9. Procédé selon les revendications 1 à 4, **caractérisé en ce qu'**on utilise en tant qu'imine tertiaire sp²-hybridée une guanidine de formule générale (IV) 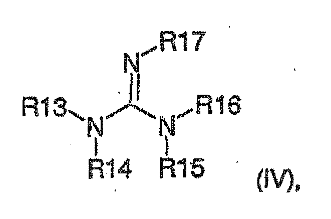 dans laquelle
les radicaux R¹³ à R¹⁷ représentent, indépendamment les uns des autres, un radical organique contenant du carbone, saturé ou insaturé, cyclique ou acyclique, aliphatique, aromatique ou araliphatique, ayant de 1 à 20 atomes de carbone, non substitué ou interrompu ou substitué par 1 à 5 hétéroatomes ou groupes fonctionnels, les radicaux R¹³ et R¹⁵ pouvant également représenter, en outre un atome d'hydrogène ; ou
chaque fois indépendamment les des autres, les radicaux R¹³ et R¹⁴ et/ou R¹⁵ et R¹⁶ représentent ensemble un radical organique à deux liaisons, contenant du carbone, saturé ou insaturé, cyclique ou acyclique, aliphatique, aromatique ou araliphatique, ayant de 1 à 30 atomes de carbone, non substitué ou interrompu ou substitué par 1 à 5 hétéroatomes ou groupes fonctionnels, et le radical restant/les radicaux restants est/sont tel(s) que défini(s) précédemment ; ou
les radicaux R¹⁴ et R¹⁵ représentent ensemble un radical organique à deux liaisons, contenant du carbone, saturé ou insaturé, cyclique ou acyclique, aliphatique, aromatique ou araliphatique, ayant de 1 à 30 atomes de carbone, non substitué ou interrompu ou substitué par 1 à 5 hétéroatomes ou groupes fonctionnels, et les radicaux restants sont tels que définis précédemment.

10. Procédé selon les revendications 1 à 9, **caractérisé en ce qu'**on utilise en tant qu'amine tertiaire sp³-hybridée ou imine tertiaire sp²-hybridée la triméthylamine, la diméthyléthylamine, la diméthyl-n-propylamine, la diéthylméthylamine, la triéthylamine, le tri-n-propylamine, la di-n-propylméthylamine, la tri-n-butylamine, la di-n-butylméthylamine, la tri-n-pentylamine, la N-méthylpipéridine, la N,N-diméthylaniline, la N-méthylmorpholine, le N-méthylimidazole, le N-éthylimidazole, le N-(1-propyl)imidazole, le N-(1-butyl)imidazole, le N-(1-hexyl)imidazole, le N-(1-octyl)imidazole, le N-(1-décyl)imidazole, le N-(1-dodécyl)imidazole, le N-(1-pentadécyl)-imidazole, la pyridine, la 2-méthylpyridine, la 3-méthylpyridine, la 4-méthylpyridine, la 2,4-diméthylpyridine, la 2,6-diméthylpyridine, la 2-éthylpyridine, la 2,6-diéthylpyridine ou la N,N,N',N',N"-pentaméthylguanidine.
